# EUROPÄISCHE PATENTSCHRIFT

(11) **EP 1 991 283 B1**
(45) Veröffentlichungstag und Bekanntmachung des Hinweises auf die Patenterteilung: **16.05.2012**
(21) Anmeldenummer: 07722994.6
(22) Anmeldetag: 02.03.2007
(51) Int. Cl.: A61L 27/34, A61L 27/50

(54) **SCHLAUCHFÖRMIGE GEFÄRBTE GEFÄßPROTHESE UND IHRE VERWENDUNG IN DER CHIRURGIE**
TUBULAR COLORED VESSEL PROSTHESIS, AND USE THEREOF IN SURGERY
PROTHÈSE VASCULAIRE COLORÉE TUBULAIRE ET UTILISATION EN CHIRURGIE

(30) Priorität: 03.03.2006 DE 102006011218
(43) Veröffentlichungstag der Anmeldung: 19.11.2008
(73) Patentinhaber: Aesculap AG, 78532 Tuttlingen (DE)
(72) Erfinder: GOLDMANN, Helmut, 78532 Tuttlingen/Donau (DE); RIMPLER, Hartmut, 10249 Berlin (DE)
(74) Vertreter: Renger, Florian
(86) Internationale Anmeldenummer: PCT/EP2007/001794
(87) Internationale Veröffentlichungsnummer: WO 2007/101613

(56) Entgegenhaltungen:
- EP-A- 1 016 422
- US-A- 3 805 301
- US-A1- 2004 253 185

## Beschreibung

Die Erfindung betrifft eine schlauchförmige gefärbte Gefäßprothese sowie ihre Verwendung in der Chirurgie.

Die üblicherweise in der chirurgischen Versorgung zum Einsatz kommenden Gefäßprothesen sind weiß oder schwarz gefärbt. Insbesondere bei textilen Gefäßprothesen wird der weiße Farbeindruck durch sogenannte Weißpigmente, beispielsweise Titandioxid, erzeugt und/oder verstärkt. Die schwarze Färbung von Gefäßprothesen wird häufig durch eine Beschichtung mit pyrolytischem Kohlenstoff erzeugt. Daneben kommen auch grau und braun gefärbte Gefäßprothesen zum Einsatz, deren Färbungen insbesondere auf entsprechende Silbersalzbeschichtungen zurückzuführen sind.

US3805301 offenbart eine schlauchförmige Prothese von Y-förmiger Gestalt.

EP1016422 offenbart ein medizinisches Implantat auf der Basis eines optisch transparenten, röntgenundurschlässigen Polymers und eines Füllmaterials mit einer röntgenundurchlässigen Komponente beschrieben.

US2004/253185 offenbart bunte Färbungen, insbesondere von rot abweichende bunte Färbungen, medizintechnischen Produkten, insbesondere Gefässprothesen.

Nachteilig bei den aus dem Stand der Technik bekannten gefärbten Gefäßprothesen ist insbesondere ihre teilweise nur mäßige dreidimensionale Erkennbarkeit für den Chirurgen, beispielsweise im Rahmen von endoskopischen Operationen und Kontrollen. Weitere Nachteile können sich insbesondere aus einer suboptimalen Nahtjustierung mit insbesondere ungleichmäßigen Stichlängen und Abständen ergeben, wodurch grundsätzlich die Risiken einer unzureichenden Verankerung der Prothesen am Implantationsort erhöht werden können.

Aufgabe der Erfindung ist es somit, eine gefärbte Gefäßprothese bereitzustellen, welche eine gegenüber den aus dem Stand der Technik bekannten Gefäßprothesen verbesserte Erkennbarkeit beim Chirurgen hervorruft und eine möglichst einfache und unkomplizierte bestimmungsgemäße Handhabung (Handling) erlaubt.

Diese Aufgabe wird erfindungsgemäß gelöst durch eine schlauchförmige Gefäßprothese mit einer Innen- und einer Außenoberfläche und einer Wandung, wobei die Gefäßprothese an der Innen- und Außenoberfläche eine Färbung aufweist, die einen Kontrast zwischen der Innen- und Außenoberfläche bewirkt. Dadurch wird eine verbesserte dreidimensionale Erkennbarkeit bewirkt, die es dem Chirurgen erleichtert, einwandfreie Nähte anzubringen. Die Erfindung ist von besonderem Vorteil bei schräg angeschnittenen Gefäßprothesen, weil in diesem Fall die Innenoberfläche besonders gut sichtbar ist.

In einer Ausführungsform der Erfindung ist die Färbung flächig und erstreckt sich insbesondere mindestens auf einen Teil der Innen- und Außenoberfläche der erfindungsgemäßen Gefäßprothese. Vorzugsweise handelt es sich bei der Färbung der erfindungsgemäßen Gefäßprothese um eine sogenannte geschlossene Färbung. Unter einer geschlossenen Färbung im Sinne der vorliegenden Erfindung soll eine Färbung verstanden werden, die sich ohne Unterbrechungen auf die gesamte Innen- und/oder Außenoberfläche der Gefäßprothese erstreckt.

In einer weiteren Ausführungsform ist die Färbung der erfindungsgemäßen Gefäßprothese deckend. Deckend im Sinne der vorliegenden Erfindung bedeutet, daß das unter der Färbung befindliche Prothesenmaterial für den Anwender nicht sichtbar ist.

In einer besonders bevorzugten Ausführungsform der erfindungsgemäßen Gefäßprothese ist die Färbung abdichtend. Unter einer abdichtenden Färbung im Sinne der vorliegenden Erfindung soll eine Färbung verstanden werden, welche die erfindungsgemäße Gefäßprothese undurchlässig macht, insbesondere für Flüssigkeiten, beispielsweise Körperflüssigkeiten. Durch die abdichtende vorzugsweise resorbierbare Färbung der erfindungsgemäßen Gefäßprothese kann in vorteilhafter Weise der Verlust von Körperflüssigkeit, vorzugsweise von Blut, bei der Implantation der Gefäßprothese vermieden werden.

Die Färbung der erfindungsgemäßen Gefäßprothese ist mit besonderem Vorteil durch eine farbige Beschichtung oder durch eine farbige Imprägnierung, vorzugsweise durch eine farbige Imprägnierung, bedingt. Die Beschichtung oder Imprägnierung der erfindungsgemäßen Gefäßprothese kann insbesondere biologische Materialien, beispielsweise Kollagen, Gelatine oder Albumin, umfassen. In einer bevorzugten Ausführungsform der erfindungsgemäßen Gefäßprothese umfasst die Beschichtung oder Imprägnierung mindestens ein Polymer, insbesondere mindestens ein Copolymer, vorzugsweise mindestens ein Terpolymer. Bei dem Polymer kann es sich insbesondere um ein sternförmiges Polymer handeln, das vorzugsweise in Gegenwart einer tri- oder tetrafunktionellen organischen Verbindung und insbesondere in Gegenwart eines Organometallkatalysators herstellbar ist. Bei der tri- oder tetrafunktionellen Verbindung kann es sich insbesondere um Glycerol, Ethantrimethylol, Propan-trimethylol, Pentaerythritol oder Triethanolamin handeln. Derartige sternförmige Polymere sind insbesondere aus der US 6,462,169 B1 bekannt, deren Inhalt durch Bezugnahme vollumfänglich zum Gegenstand der Offenbarung der vorliegenden Erfindung gemacht wird.

Mit besonderem Vorteil umfaßt die Beschichtung oder Imprägnierung ein synthetisches resorbierbares Polymer. In einer besonders bevorzugten Ausführungsform der erfindungsgemäßen Gefäßprothese umfaßt die Beschichtung oder Imprägnierung ein Polymer auf der Basis von Glykolid, Lactid, ε-Caprolacton und/oder Trimethylencarbonat.

In einer weiteren Ausführungsform der erfindungsgemäßen Gefäßprothese weist die Färbung eine Leuchtfarbe auf. Dies kann insbesondere den Kontrast zwischen der Innen- und Außenoberfläche der erfindungsgemäßen Gefäßprothese erhöhen und somit insbesondere ihre Handhabung, vorzugsweise in Bezug auf ihre dreidimensionale Erkennbarkeit, bei der Implantation verbessern. Weiterhin ist es erfindungsgemäß bevorzugt vorgesehen, daß die Färbung der Gefäßprothese nicht spiegelnd ist. Die nicht spiegelnden Eigenschaften der erfindungsgemäßen Gefäßprothese erlauben insbesondere ein sichere und genaue Nahtjustierung beim Ligieren der erfindungsgemäßen Gefäßprothese mit natürlichen Gefäßorganen, vorzugsweise mit Blutgefäßen.

In einer besonders bevorzugten Ausführungsform der Erfindung ist die Gefäßprothese mehrfarbig, vorzugsweise zweifarbig.

In einer weiteren besonders bevorzugten Ausführungsform der erfindungsgemäßen Gefäßprothese weisen die innen- und Außenoberfläche jeweils eine Färbung auf, wobei die Innenoberfläche der Gefäßprothese eine andere Färbung besitzt als die Außenoberfläche. Erfindungsgemäß ist es insbesondere vorgesehen, daß die Färbungsunterschiede zwischen der Innen- und der Außenoberfläche der Gefäßprothese auf unterschiedlichen, insbesondere stark verschiedenen, Färbungsintensitäten derselben Farbe beruhen. Die unterschiedliche Färbung zwischen der Innen- und Außenoberfläche kann in besonderem Maße darauf zurückgeführt werden, daß die für die Färbung verantwortlichen Farbstoffe, insbesondere die farbige Beschichtung oder die farbige Imprägnierung, nicht vollständig durch die Wandung in das Innere der Gefäßprothese eindringen. Bezüglich weiterer Eigenschaften der Farbstoffe wird auf die folgende Beschreibung verwiesen. Auf diese Weise wird mit besonderem Vorteil ein Kontrast zwischen der Innen- und Außenoberfläche der erfindungsgemäßen Gefäßprothese erzeugt, welcher insbesondere zu einer Verbesserung des dreidimensionalen Eindrucks der Gefäßprothese führt. Durch die verbesserte Kontrastdarstellung zwischen der Innen- und der Außenoberfläche der erfindungsgemäßen Gefäßprothese können insbesondere Fehler bei der Implantation der Gefäßprothese, beispielsweise eine ungleichmäßige Nahtjustierung, besser erkannt und gegebenenfalls korrigiert werden.

Weiterhin kann die erfindungsgemäße Gefäßprothese an den Enden der Innen- und/oder Außenoberfläche, vorzugsweise an den Enden der Außenoberfläche, gefärbt sein. Mit Vorteil weist die erfindungsgemäße Gefäßprothese an ihren Enden unterschiedliche Färbungen auf. Gegebenenfalls kann die erfindungsgemäße Gefäßprothese zwischen ihren gefärbten Prothesenenden weitere Färbungen aufweisen. Vorzugsweise sind die Färbungen als eingefärbte Ringe ausgebildet, welche insbesondere in gleichmäßigen Abständen auf der Innen- und/oder Außenoberfläche, vorzugsweise auf der Außenoberfläche, der erfindungsgemäßen Gefäßprothese angeordnet sind. Somit können beispielsweise unterschiedliche Durchmesser bei der erfindungsgemäßen Gefäßprothese, die insbesondere in konischer Form vorliegen kann, besser visualisiert werden.

In einer insbesondere bevorzugten Ausführungsform ist die erfindungsgemäße Gefäßprothese an der Innenoberfläche ungefärbt (weiß) und an der Außenoberfläche gefärbt. Dies kann in vorteilhafter Weise eine Kontrastverstärkung zwischen der Innen- und Außenoberfläche der erfindungsgemäßen Gefäßprothese bewirken.

Die Färbung der erfindungsgemäßen Gefäßprothese ist bevorzugt durch bioverträgliche Farbstoffe verursacht. Bei den Farbstoffen kann es sich insbesondere um Farbstoffe für Nahtmaterialien, insbesondere um Farbstoffe für resorbierbare Nahtmaterialien, handeln.

In einer Ausführungsform der erfindungsgemäßen Gefäßprothese ist die Färbung auf wasserlösliche Farbstoffe, insbesondere auf Lebensmittelfarbstoffe, zurückzuführen. Solche Farbstoffe eignen sich besonders für Beschichtungs- oder Imprägnierungsmittel auf wäßriger Basis.

In einer besonders bevorzugten Ausführungsform der Erfindung ist die Färbung der Gefäßprothese durch mindestens einen in organischen Lösungsmitteln, insbesondere in Ketonen, löslichen Farbstoff bedingt. Solche Farbstoffe eignen sich besonders für Beschichtungs- oder Imprägnierungsmittel auf Basis von in organischen Lösungsmitteln löslichen Beschichtungs- oder Imprägnierungsmitteln auf Polymerbasis. Bei den organischen Lösungsmitteln handelt es sich bevorzugt um 3-Pentanon und/oder Aceton, vorzugsweise um 3-Pentanon. Erfindungsgemäß ist es besonders bevorzugt, daß es sich bei dem in organischen Lösungsmitteln löslichen Farbstoff um D & C (Drug & Cosmetic) No. 2 und/oder D & C (Drug & Cosmetic) No. 6 handelt.

Weiterhin kann die Färbung der erfindungsgemäßen Gefäßprothese durch mehrere Farbstoffe, insbesondere durch eine Farbstoffmischung, verursacht sein.

Erfindungsgemäß ist es besonders bevorzugt, daß die Färbung der Gefäßprothese auf mindestens einer von rot abweichenden bunten Farbe beruht. Dies ist besonders vorteilhaft, da auf diese Weise ein Kontrast zu der roten Farbe des menschlichen und/oder tierischen Blutes erzeugt wird, wodurch insbesondere Fehler, beispielsweise durch ungenaue Nahtjustierungen, vermieden werden können. Dies ermöglicht insgesamt eine deutliche Erleichterung der Handhabung der erfindungsgemäßen Gefäßprothese für den Chirurgen.

In einer vorteilhaften Ausführungsform der erfindungsgemäßen Gefäßprothese umfasst die Färbung mindestens eine Farbe aus der Gruppe violett, blau, grün und gelb. Erfindungsgemäß ist es besonders bevorzugt, daß die Außenoberfläche der Gefäßprothese blau bis violett gefärbt ist.' Vorzugsweise ist die Außenoberfläche der erfindungsgemäßen Gefäßprothese blau bis violett gefärbt und die Innenoberfläche schwächer blau bis violett gefärbt. In einer weiteren bevorzugten Ausführungsform ist die Außenoberfläche der erfindungsgemäßen Gefäßprothese blau bis violett gefärbt und die Innenoberfläche ungefärbt.

In einer anderen bevorzugten Ausführungsform der Erfindung ist die Außenoberfläche der Gefäßprothese grün gefärbt. Vorzugsweise ist die Außenoberfläche der erfindungsgemäßen Gefäßprothese grün gefärbt und die Innenoberfläche schwächer grün gefärbt. Erfindungsgemäß ist es besonders bevorzugt, daß die Außenoberfläche der Gefäßprothese grün gefärbt und die Innenoberfläche ungefärbt ist.

Die erfindungsgemäße Gefäßprothese weist vorzugsweise eine textile Konstruktion auf. Mit Vorteil handelt es sich bei der erfindungsgemäßen Gefäßprothese um eine gewirkte oder gewebte Gefäßprothese. Die Gefäßprothese gemäß der Erfindung kann nach speziellen Wirk- oder Webtechniken hergestellt sein. Die Wirk- und Webtechniken sind dem Fachmann allgemein bekannt, so daß auf eine ausführlichere Beschreibung verzichtet wird.

Weiterhin kann die erfindungsgemäße Gefäßprothese als sogenannte non-woven Struktur, vorzugsweise aus expandiertem Polytetrafluorethylen (ePTFE) oder Polyurethan, ausgebildet sein. Bevorzugt ist die erfindungsgemäße Gefäßprothese als Vliesstruktur, vorzugsweise als Sprühvliesstruktur, insbesondere aus Polyurethan, ausgebildet.

Die erfindungsgemäße Gefäßprothese ist vorzugsweise aus multifilamenten Fäden gebildet. Multifilamente Gefäßprothesen verfügen über eine aufgrund der zwischen den Einzelfilamenten auftretenden Kapillarkräfte optimale Aufnahmekapazität für Flüssigkeiten, insbesondere für Körperflüssigkeiten, vorzugsweise für Blut. Dies kann im Fall von nicht imprägnierten Gefäßprothesen zur Abdichtung durch Blutgerinnung während der Implantation besonders vorteilhaft sein.

Bei den Wandungsmaterialien der erfindungsgemäßen Gefäßprothese kann es sich insbesondere um Polypropylen, Polyester, beispielsweise Polyethylenterephthalat (PET), expandiertes Polytetrafluorethylen (ePTFE) oder Polyurethan handeln Polyurethan, insbesondere lineares Polyurethan, vorzugsweise lineares aliphatisches Polyurethan, ist wegen seiner hohen Elastizität und insbesondere geringen materialbedingten Thrombogenität in besonderem Maße als Wandungsmaterial für die erfindungsgemäße Gefäßprothese geeignet.

Die vorliegende Erfindung betrifft weiterhin ein Verfahren zur Herstellung der erfindungsgemäßen Gefäßprothese, umfassend den Schritt:
- Auftrag mindestens einer gefärbten Lösung oder mindestens einer gefärbten Suspension auf eine Gefäßprothese oder auf Wandungsmaterialien der Gefäßprothese.

In einer bevorzugten Ausführungsform des erfindungsgemäßen Verfahrens werden die Enden der Gefäßprothese vor Auftrag der gefärbten Lösung oder gefärbten Suspension dicht verschlossen. Auf diese Weise kann mit besonderem Vorteil eine Gefäßprothese hergestellt werden, die einen Kontrast zwischen der Innen- und der Außenoberfläche aufweist. Je nach Dichtigkeit des Verschlusses und insbesondere der Prothesenwandung kann eine Gefäßprothese hergestellt werden, deren Innenoberfläche ungefärbt ist oder deren Innenoberfläche eine andere Färbung besitzt als die Außenoberfläche der Gefäßprothese. Vorzugsweise werden die Materialien für den Verschluß der Prothesenenden und insbesondere für die Prothesenwandung so gewählt, dass die Gefäßprothese nach Einfärbung eine Innenoberfläche aufweist, die eine andere Färbung besitzt als die Außenoberfläche der Gefäßprothese. Bevorzugt werden Verschluß- und insbesondere Wandungsmaterialien verwendet, die einen vollständigen Durchtritt der gefärbten Lösung oder gefärbten Suspension durch die Abdichtung der Prothesenenden und insbesondere durch die Wandung der Gefäßprothese verhindern. Eine Vorimprägnierung, insbesondere der Innenoberfläche, mit farblosen oder andersfarbigen Imprägnierungsmitteln kann vorteilhaft sein.

In einer insbesondere bevorzugten Ausführungsform des erfindungsgemäßen Verfahrens werden die offenen Enden der Gefäßprothese für die Färbung dicht verschlossen. Auf diese Weise können mit besonderem Vorteil die unterschiedlichen Färbungen zwischen der Innen- und Außenoberfläche der Gefäßprothese hergestellt werden. Bezüglich weiterer Einzelheiten hierzu wird auf die bisherige Beschreibung verwiesen.

In einer weiteren Ausführungsform des erfindungsgemäßen Verfahrens wird eine gefärbte Beschichtungs- oder gefärbte Imprägnierlösung auf die Gefäßprothese aufgetragen. In einer anderen Ausführungsform wird eine gefärbte Beschichtungs- oder gefärbte Imprägniersuspension auf die Gefäßprothese aufgetragen.

Weiterhin kann die gefärbte Lösung oder gefärbte Suspension auf eine bereits imprägnierte oder beschichtete Gefäßprothese aufgetragen werden, wobei die Gefäßprothese vorzugsweise farblos beschichtet oder imprägniert ist.

Mit besonderem Vorteil werden zur Färbung der Gefäßprothese und/ oder ihrer Wandungsmaterialien gefärbte Kollagen-, Gelatine- oder Albuminsuspensionen eingesetzt. Alternativ dazu können mit Vorteil farbige Lösungen von Polymeren in organischen Lösungsmitteln verwendet werden. Vorzugsweise werden als organische Lösungsmittel Ketone, vorzugsweise 3-Pentanon und/oder Aceton, verwendet. Die Farbigkeit der Polymerlösungen wird mit besonderem Vorteil durch die Zugabe von in organischen Lösungsmitteln löslichen Farbstoffen, vorzugsweise D & C (Drug & Cosmetic) No. 2 und/oder D & C (Drug & Cosmetic) No. 6, erreicht. Mit besonderem Vorteil werden farbige Polymerlösungen aus biologisch resorbierbaren Poymeren verwendet, wobei bevorzugt Copolymerlösungen, mit Vorteil Terpolymerlösungen, insbesondere Tetrapolymere, auf der Basis von Glykolid, Lactid, ε-Caprolacton und/oder Trimethylencarbonat zur Färbung eingesetzt werden. Bezüglich weiterer Einzelheiten wird auf die bisherige Beschreibung verwiesen.

In einer bevorzugten Ausführungsform des erfindungsgemäßen Verfahrens wird der Auftrag der gefärbten Lösung oder gefärbten Suspension durch Besprühen vorgenommen.

In einer anderen insbesondere bevorzugten Ausführungsform des erfindungsgemäßen Verfahrens wird der Auftrag der gefärbten Lösung oder gefärbten Suspension durch Eintauchen der Gefäßprothese und/oder ihrer Wandungsmaterialien in die gefärbte Lösung oder gefärbte Suspension vorgenommen. Ebenso ist es erfindungsgemäß möglich, daß die Gefäßprothese mit der gefärbten Lösung oder gefärbten Suspension übergossen wird.

In einer weiteren Ausführungsform des erfindungsgemäßen Verfahrens wird die gefärbte Lösung oder gefärbte Suspension von innen durch die Prothesenwand gepreßt, vorzugsweise unter Ausübung von Druck. Diese Vorgehensweise ist besonders zur Einfärbung von Prothesen mit ei ner non-woven-Struktur bevorzugt. Im Falle der Färbung der Gefäßprothese mit einer gefärbten Polymerlösung oder -suspension kann die Innenwand der Gefäßprothese zur Entfernung der nach Einfärbung der Gefäßprothese an der Innenwand haftenden gefärbten Polymere mit organischen Lösungsmitteln, insbesondere mit Ketonen, vorzugsweise mit 3-Pentanon und/oder Aceton, gespült werden.

In einer anderen besonders bevorzugten Ausführungsform des erfindungsgemäßen Verfahrens werden die Farbstoffe in die Polymere eingearbeitet. Vorzugsweise werden die Farbstoffe zu einer Polymerschmelze hinzugegeben. Die eingefärbte Polymerschmelze wird nach Abkühlung bevorzugt granuliert und vorzugsweise zu einem ungefärbten Granulat hinzugegeben.

Bei Prothesen aus gesprühten Schichten, beispielsweise aus einem Sprühvlies, kann mit verschieden farbigen Polymerlösungen zeitlich versetzt gearbeitet werden. So wird ein Aufbau aus verschieden gefärbten Schichten erhalten.

Zur Herstellung der erfindungsgemäßen Gefäßprothese kann es insbesondere vorgesehen sein; daß der Auftrag auf eine ungefärbte oder gefärbte Gefäßprothese vorgenommen wird, wobei für den Auftrag bevorzugt eine ungefärbte Gefäßprothese verwendet wird.

Es ist weiterhin möglich, textile Gefäßprothesen unter Verwendung von zwei verschieden gefärbten Fadensystemen herzustellen, wobei ein Fadensystem, vorzugsweise aus weißen Fäden, vorzugsweise an der Gefäßprotheseninnenseite liegt, und das andere Fadensystem, vorzugsweise aus bunten Fäden, an der Gefäßprothesenaußenseite.

Schließlich betrifft die Erfindung die Verwendung der erfindungsgemäßen Gefäßprothese zum Ersatz oder zur Überbrückung von Blutgefäßen. Bezüglich weiterer Einzelheiten wird auf die obige Beschreibung verwiesen.

Die erfindungsgemäße Gefäßprothese zeichnet sich durch einen gegenüber den aus dem Stand der Technik bekannten Gefäßprothesen deutlich verbesserten Kontrast zwischen der Innen- und Außenoberfläche aus. Hierdurch wird insbesondere die dreidimensionale Erkennbarkeit für den Chirurgen deutlich verbessert. Der bei der erfindungsgemäßen Gefäßprothese gegenüber dem Stand der Technik deutlich verstärkte Kontrast bewirkt insbesondere eine genaue und vorzugsweise gleichmäßige Nahtjustierung. Dies ist in Bezug auf die Ligierung der erfindungsgemäßen Gefäßprothese mit natürlichen Gefäßorganen, vorzugsweise Blutgefäßen, von besonderer Bedeutung.

### Figurenbeschreibung

Figur 1:
   Es ist eine schräg angeschnittene erfindungsgemäße Gefäßprothese 1 gezeigt, deren Wandung 2 eine Innenoberfläche 3 aufweist, die eine andere Färbung besitzt als die Außenoberfläche 4. Die unterschiedlichen Färbungen der erfindungsgemäßen Gefäßprothese sind durch die unterschiedlichen Linienmuster, welche die Innenoberfläche 3 und Außenoberfläche 4 der erfindungsgemäßen Gefäßprothese 1 abbilden, zum Ausdruck gebracht.
Figur 2:
   Es ist eine schräg angeschnittene erfindungsgemäße Gefäßprothese 1 gezeigt, deren Wandung 2 an der Innenoberfläche 3 ungefärbt ist und
   an der Außenoberfläche 4 eine blauviolette Färbung aufweist. Die blauviolette Färbung der Außenoberfläche 4 der erfindungsgemäßen Gefäßprothese 1 ist durch ein im Wesentlichen rechtwinklig verlaufendes Linienmuster zum Ausdruck gebracht.
Figur 3:
   Es ist eine schräg angeschnittene erfindungsgemäße Gefäßprothese 1 gezeigt, deren Wandung 2 an der Innenoberfläche 3 ungefärbt ist und
   an der Außenoberfläche 4 grün gefärbt ist. Die grüne Färbung der erfindungsgemäßen Gefäßprothese 1 an der Außenoberfläche 4 ist durch ein im Wesentlichen rautenförmiges Linienmuster zum Ausdruck gebracht.
Figur 4:
   Es sind die Ergebnisse der Kontrastmessung verschiedener Gefäßprothesen graphisch in Form eines Histogramms wiedergegeben. Auf der Ordinate der Graphik ist der Pegel der Luminanz [mV] angegeben. Auf der Abszisse sind verschiedene gemessene Gefäßprothesen 1 bis 5 aufgeführt. Die hellen Balken spiegeln jeweils den gemessenen Luminanzpegel an der Außenoberfläche der jeweiligen Gefäßprothese wider. Die dunklen Balken spiegeln jeweils den gemessenen Luminanzpegel an der Innenoberfläche der jeweiligen Gefäßprothese wider. Im Falle der Gefäßprothese 1 handelt es sich um eine mit Silberacetat gefärbte Gefäßprothese. Die Gefäßprothese 2 ist eine mit Silber beschichtete Gefäßprothese. Die Gefäßprothese 3 ist eine unbeschichtete farblose Gefäßprothese. Bei der Gefäßprothese 4 handelt es sich um eine farblos beschichtete Gefäßprothese. Die soeben aufgeführten und näher bezeichneten Gefäßprothesen 1 bis 4 sind aus dem Stand der Technik bekannt. Demgegenüber handelt es sich bei der Gefäßprothese 5 um eine erfindungsgemäß gefärbte und Polymer beschichtete Gefäßprothese, welche an der Außenoberfläche blau gefärbt und an der Innenoberfläche deutlich schwächer blau gefärbt oder farblos ist. Der Kontrast bei den jeweiligen Gefäßprothesen ergibt sich aus dem Unterschied der gemessenen Luminanzpegel an der Innen- und Außenoberfläche der jeweiligen Gefäßprothesen.

Weitere Merkmale und Einzelheiten der Erfindung ergeben sich aus der nachfolgenden Beschreibung einer bevorzugten Ausführungsform in Form eines Beispiels. Dabei können die einzelnen Merkmale jeweils für sich alleine oder zu mehreren in Kombination miteinander verwirklicht sein. Das Beispiel dient lediglich der Erläuterung der vorliegenden Erfindung, die in keiner Weise darauf beschränkt sein soll.

### Beispiel:

### 1. Herstellung einer gefärbten Vlies-Gefäßprothese

Zur Herstellung der Prothesenwandung wird eine Lösung von linearem Polyurethan in Chloroform auf einen rotierbaren Stab aufgesprüht. Anschließend wird eine mit D & C violett No.2 gefärbte Polymerlösung auf die aufgesprühte Polyurethanschicht ebenfalls durch Sprühen aufgetragen. Auf diese Weise wird eine Vlies-Gefäßprothese erhalten, welche an ihrer Außenoberfläche violett gefärbt ist, wohingegen ihre Innenoberfläche eine im Vergleich zur Außenoberfläche deutlich schwächere Violettfärbung aufweist oder ungefärbt ist.

### 2. Herstellung einer gefärbten und gewirkten Gefäßprothese

Eine aus Polyethylenterephtalat (PET) gewirkte Gefäßprothese wird mit einer Gelatinesuspension in bekannter Weise beschichtet. Dabei werden die Gefäßprothesen auf Metallstäbe, die im Durchmesser und der Form der jeweiligen Prothese angepasst sind, vor dem Beschichtungsprozess aufgezogen und an den Enden beispielsweise mit Kabelbinder fixiert. Die Gelatinesuspension enthält einen wasserlöslichen blauen Farbstoff, der für eine Färbung der Gefäßprothese entsprechend Beispiel 1 verantwortlich ist (stark unterschiedliche Färbungsintensitäten derselben Farbe an der Innen- und Außenoberfläche der Gefäßprothese). Anschließend kann die Gelatine mit Diisocyanat vernetzt und in üblicher Weise weiterbehandelt werden.

### 3. Herstellung einer farbigen und gewirkten Gefäßprothese unter Verwendung von zwei Fadensystemen

Für die Herstellung der Gefäßprothese werden eine zweifonturige Kettenwirkmaschine sowie ein weißes und blaues Fadensystem verwendet. Die Kettenwirkmaschine ist so eingestellt, dass die weißen Fäden überwiegend auf der Innenseite des Gewirks liegen und die blauen Fäden überwiegend auf der Außenseite des Gewirks. Durch die geringen Anteile der blauen Fäden, welche an der Innenseite des Gewirks sichtbar sind, ist die hergestellte Gefäßprothese an der Innenseite nur leicht gefärbt, so dass ein starker Unterschied in der Färbungsintensität derselben Farbe im Vergleich zur Außenseite der Gefäßprothese vorliegt. Auf diese Weise ist ebenfalls ein eindeutiger Kontrastunterschied zwischen der Innen- und Außenoberfläche der Gefäßprothese erzielbar.

### 4. Kontrastmessung von Gefäßprothesen:

Im Rahmen der vorliegenden Messreihe wurde der Kontrast der folgenden Gefäßprothesen bestimmt:
1. Gefäßprothese 1
2. Gefäßprothese 2
3. Gefäßprothese 3
4. Gefäßprothese 4
5. Gefäßprothese 5

Für die Messung des Kontrastes der Gefäßprothesen 1 bis 5 wurde eine 180 W Xenon-Lichtquelle mit angeschlossener Endoskopiekamera mit 1-CCD (Charged Coupled Devices) sowie ein sogenannter Vektorskop verwendet. Die Kontrastmessung erfolgte anhand des Pegels [mV] der Luminanz (Bildhelligkeit bzw. -intensität). Zur Kalibrierung wurde diese optische Versuchsanordnung mit angeschlossener Endoskopiekamera und angeschlossener Beleuchtung auf eine weiße Fläche gerichtet. Die Beleuchtungsstärke der Xenon-Lichtquelle wurde so eingestellt, dass ein Luminanzpegel von 760 mV erreicht wurde.

Zur Messung des Kontrasts wurden die Gefäßprothesen 1 bis 5 jeweils auf eine schwarze Fläche gelegt. Anschließend wurde die Xenon-Lichtquelle mit angeschlossener Endoskopiekamera jeweils auf die Gefäßprothese gerichtet.

Die Kontrastmessung der Gefäßprothesen führte zu den folgenden Messergebnissen:

| Probe | außen | innen |
|---|---|---|
| Gefäßprothese 1 | 434 | 608 |
| Gefäßprothese 2 | 684 | 751 |
| Gefäßprothese 3 | 751 | 760 |
| Gefäßprothese 4 | 750 | 739 |
| Gefäßprothese 5 | 311 | 725 |

### Ergebnis:

Die Messergebnisse zeigen deutlich, dass die erfindungsgemäße Gefäßprothese 5 einen gegenüber den aus dem Stand der Technik bekannten Gefäßprothesen deutlich verbesserten Kontrast zwischen der Innen- und Außenoberfläche aufweist.

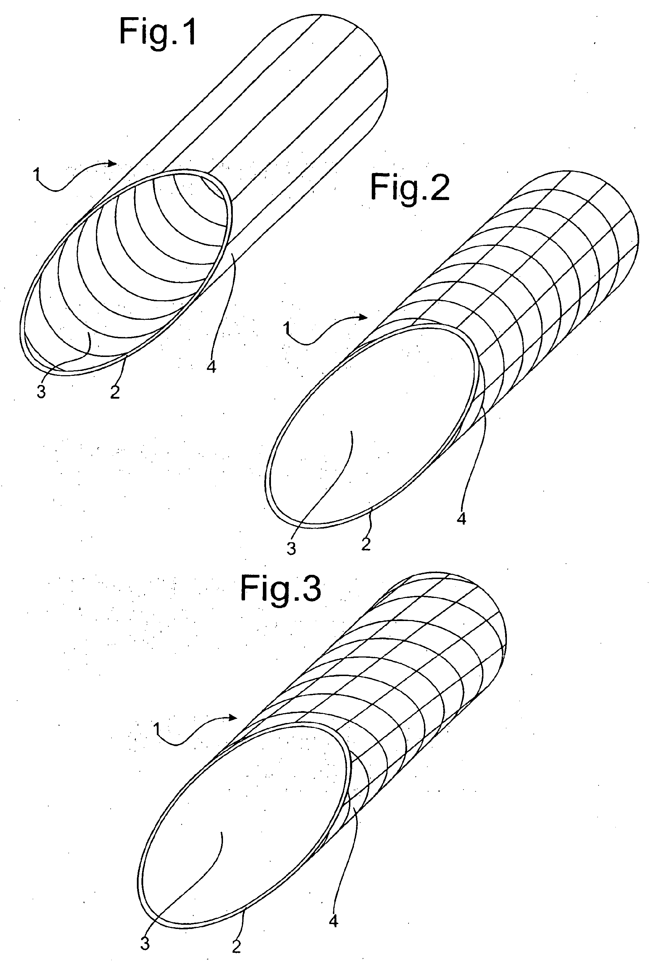

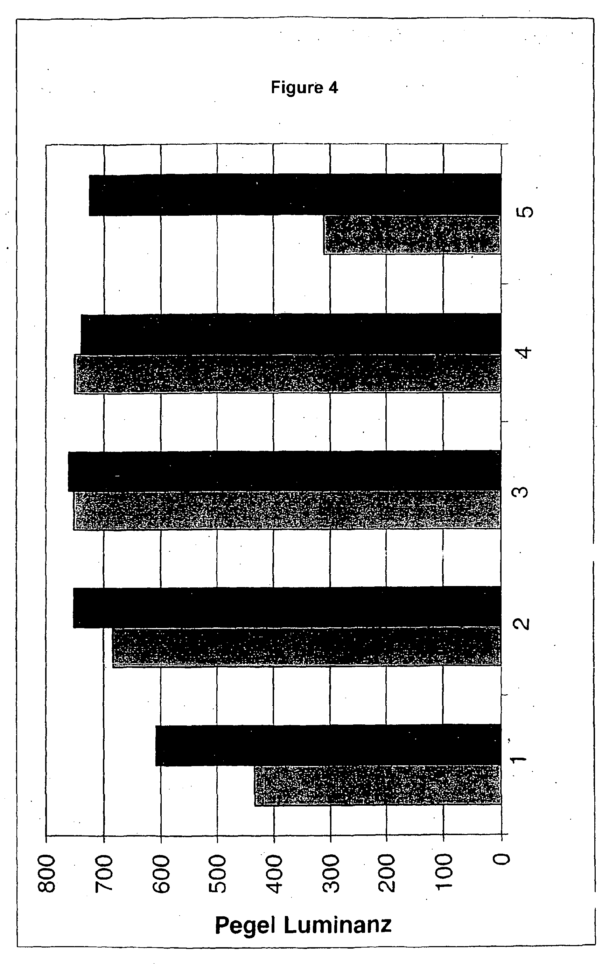

## Patentansprüche

1. Schlauchförmige Gefäßprothese mit einer Innen- und einer Außenoberfläche und einer Wandung, wobei die Gefäßprothese an der Innen- und Außenoberfläche jeweils eine Färbung aufweist, die einen Kontrast zwischen der Innen- und Außenoberfläche bewirkt, **dadurch gekennzeichnet, dass** die Innenoberfläche eine andere Färbung besitzt als die Außenoberfläche der Gefäßprothese und die Färbung auf mindestens einer von rot abweichenden bunten Farbe beruht.

2. Gefäßprothese nach Anspruch 1, **dadurch gekennzeichnet, daß** sie geschlossen gefärbt ist.

3. Gefäßprothese nach Anspruch 1 oder 2, **dadurch gekennzeichnet, daß** die Färbung abdichtend ist.

4. Gefäßprothese nach einem der vorhergehenden Ansprüche, **dadurch gekennzeichnet, daß** die Färbung eine farbige Beschichtung oder farbige Imprägnierung ist.

5. Gefäßprothese nach Anspruch 4, **dadurch gekennzeichnet, daß** die Beschichtung oder Imprägnierung ein Polymer, vorzugsweise ein synthetisches resorbierbares Polymer, vorzugsweise auf der Basis von Glykolid, Lactid, ε-Caprolacton und/oder Trimethylencarbonat, umfaßt.

6. Gefäßprothese nach einem der vorhergehenden Ansprüche, **dadurch gekennzeichnet, daß** die Färbung nicht spiegelnd ist.

7. Gefäßprothese nach einem der vorhergehenden Ansprüche, **dadurch gekennzeichnet, daß** sie mehrfarbig, vorzugsweise zweifarbig, ist.

8. Gefäßprothese nach einem der vorhergehenden Ansprüche, **dadurch gekennzeichnet, daß** die Färbung durch mindestens einen in organischen Lösungsmitteln, insbesondere in Ketonen, löslichen Farbstoff bedingt ist.

9. Gefäßprothese nach einem der vorhergehenden Ansprüche, **dadurch gekennzeichnet, daß** die Färbung mindestens eine Farbe aus der Gruppe violett, blau, grün und gelb umfaßt.

10. Gefäßprothese nach einem der vorhergehenden Ansprüche, **dadurch gekennzeichnet, daß** die Außenoberfläche blau bis violett gefärbt ist.

11. Gefäßprothese nach einem der Ansprüche 1 bis 9, **dadurch gekennzeichnet, daß** die Außenoberfläche grün gefärbt ist.

12. Gefäßprothese nach einem der vorhergehenden Ansprüche zum Ersatz oder zur Überbrückung von Blutgefäßen.

## Claims

1. Tubular vessel prosthesis with an inner surface and an outer surface and a wall, the vessel prosthesis having in each case a coloration on the inner surface and outer surface which brings about a contrast between the inner surface and outer surface, **characterized in that** the inner surface has a coloration different from the outer surface of the vessel prosthesis and the coloration derives from at least one bright color different from red.

2. Vessel prosthesis according to Claim 1, **characterized in that** it has a continuous coloration.

3. Vessel prosthesis according to Claim 1 or 2, **characterized in that** the coloration is sealing.

4. Vessel prosthesis according to any of the preceding claims, **characterized in that** the coloration is a colored coating or colored impregnation.

5. Vessel prosthesis according to Claim 4, **characterized in that** the coating or impregnation comprises a polymer, preferably a synthetic absorbable polymer, preferably based on glycolide, lactide, ε-caprolactone and/or trimethylene carbonate.

6. Vessel prosthesis according to any of the preceding claims, **characterized in that** the coloration is non-reflective.

7. Vessel prosthesis according to any of the preceding claims, **characterized in that** it is multicolored, preferably bicolored.

8. Vessel prosthesis according to any of the preceding claims, **characterized in that** the coloration is caused by at least one dye which is soluble in organic solvents, in particular in ketones.

9. Vessel prosthesis according to any of the preceding claims, **characterized in that** the coloration comprises at least one color from the group of violet, blue, green and yellow.

10. Vessel prosthesis according to any of the preceding claims, **characterized in that** the outer surface has a blue to violet coloration.

11. Vessel prosthesis according to any of Claims 1 to 9, **characterized in that** the outer surface has a green coloration.

12. Vessel prosthesis according to any of the preceding claims for replacing or for bridging over blood vessels.

## Revendications

1. Prothèse vasculaire en forme de tuyau flexible, dotée d'une surface intérieure, d'une surface extérieure et d'une paroi, la prothèse vasculaire présentant sur sa surface intérieure et sur sa surface extérieure à chaque fois une coloration qui a pour effet un contraste entre la surface intérieure et la surface extérieure,
**caractérisée en ce que**
la surface intérieure présente une autre coloration que la surface extérieure de la prothèse vasculaire et
**en ce que** la coloration est basée sur au moins un colorant différent du rouge.

2. Prothèse vasculaire selon la revendication 1, **caractérisée en ce qu'**elle présente une coloration fermée.

3. Prothèse vasculaire selon la revendication 1 ou 2, **caractérisée en ce que** la coloration a un effet d'étanchéité.

4. Prothèse vasculaire selon l'une des revendications précédentes, **caractérisée en ce que** la coloration est un revêtement coloré ou une imprégnation colorée.

5. Prothèse vasculaire selon la revendication 4, **caractérisée en ce que** le revêtement ou l'imprégnation comporte un polymère, de préférence un polymère synthétique résorbable, de préférence à base de glycolide, de lactide, d'ε-caprolactone et/ou de carbonate de triméthylène.

6. Prothèse vasculaire selon l'une des revendications précédentes, **caractérisée en ce que** la coloration n'est pas réfléchissante.

7. Prothèse vasculaire selon l'une des revendications précédentes, **caractérisée en ce qu'**elle présente plusieurs couleurs et de préférence deux couleurs.

8. Prothèse vasculaire selon l'une des revendications précédentes, **caractérisée en ce que** la coloration est provoquée par au moins un colorant soluble dans des solvants organiques, et en particulier dans les cétones.

9. Prothèse vasculaire selon l'une des revendications précédentes, **caractérisée en ce que** la coloration comporte au moins une couleur de l'ensemble constitué du violet, du bleu, du vert et du jaune.

10. Prothèse vasculaire selon l'une des revendications précédentes, **caractérisée en ce que** la surface extérieure est colorée en bleu à violet.

11. Prothèse vasculaire selon l'une des revendications 1 à 9, **caractérisée en ce que** la surface extérieure est colorée en vert.

12. Prothèse vasculaire selon l'une des revendications précédentes, pour le remplacement ou le pontage de vaisseaux sanguins.
